(19) Europäisches Patentamt
European Patent Office
Office européen des brevets

(11) EP 4 129 678 A1

(12) **EUROPEAN PATENT APPLICATION**
published in accordance with Art. 153(4) EPC

(43) Date of publication:
**08.02.2023 Bulletin 2023/06**

(21) Application number: **21779058.3**

(22) Date of filing: **24.03.2021**

(51) International Patent Classification (IPC):
***B32B 27/40*** (1968.09) ***A61F 13/51*** (2000.01)
***A61L 15/24*** (1990.01) ***A61L 15/26*** (1990.01)
***A61L 15/42*** (1990.01) ***B32B 5/02*** (1968.09)
***B32B 27/12*** (1968.09) ***B32B 27/32*** (1968.09)

(52) Cooperative Patent Classification (CPC):
**A61F 13/51; A61L 15/24; A61L 15/26; A61L 15/42; B32B 5/02; B32B 27/12; B32B 27/32; B32B 27/40**

(86) International application number:
**PCT/JP2021/012252**

(87) International publication number:
**WO 2021/200477 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2020 JP 2020063589**

(71) Applicant: **Mitsui Chemicals, Inc.**
**Tokyo 105-7122 (JP)**

(72) Inventors:
- **YAMASAKI, Satoshi**
  **Sodegaura-shi, Chiba 299-0265 (JP)**
- **ICHIKAWA, Taro**
  **Sodegaura-shi, Chiba 299-0265 (JP)**

(74) Representative: **Hoffmann Eitle**
**Patent- und Rechtsanwälte PartmbB**
**Arabellastraße 30**
**81925 München (DE)**

(54) **LAMINATE SHEET, SANITARY MATERIAL, MEDICAL MATERIAL, AND METHOD FOR MANUFACTURING LAMINATE SHEET**

(57) A laminate sheet 1 includes a water-vapor permeable polyurethane film 2 having a hard segment phase and a first spunbond nonwoven fabric 3 disposed at one side in a thickness direction of the water-vapor permeable polyurethane film 2. The first spunbond nonwoven fabric 3 contains stretchable fibers containing a thermoplastic polyurethane and non-stretchable fibers containing a polyolefin. The melting point of the hard segment phase is 65°C or more and 140°C or less.

FIG. 1

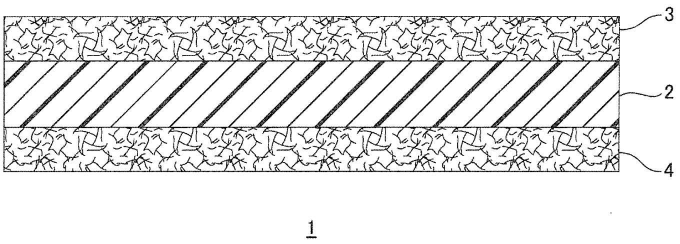


EP 4 129 678 A1

## Description

The present invention relates to a laminate sheet, a sanitary material, a medical material, and a method for manufacturing a laminate sheet.

BACKGROUND ART

Sanitary materials such as paper diapers are required to suppress the passing of liquid water and to have excellent stretchability and excellent water-vapor permeability. It has been considered to use a laminate sheet of a water-vapor permeable polyurethane film and a stretchable nonwoven fabric laminated on the film for such sanitary materials.

As such a laminate sheet, for example, a stretchable, water-vapor permeable, waterproof sheet has been proposed (for example, Patent document 1 (Example 1) below). The sheet includes a water-vapor permeable polyurethane film formed of a polyurethane resin and a nonwoven fabric made of polypropylene, where the polyurethane resin is a reaction product of an oxyalkylene polyol, 1,4-butanediol that is a chain extender, and diphenylmethane diisocyanate; the oxyalkylene polyol is obtained by the addition polymerization of 90% by weight of ethylene oxide and 10% by weight of propylene oxide; and the nonwoven fabric is laminated on the water-vapor permeable polyurethane film.

Citation List

Patent Document

Patent Document 1: Japanese Unexamined Patent Publication No. H7-70936

SUMMARY OF THE INVENTION

PROBLEM TO BE SOLVED BY THE INVENTION

However, there is a limit to the improvement of the stretchability of the stretchable water-vapor permeable sheet of Patent Document 1. Further, the nonwoven fabric of the stretchable water-vapor permeable sheet of Patent Document 1 has insufficient adhesion to the water-vapor permeable polyurethane film. Thus, the nonwoven fabric may peel from the water-vapor permeable polyurethane film. Furthermore, the reduction in tackiness properties (stickiness) of the laminate sheet is required in view of the feel of the sheet when the laminate sheet is in contact with a human body.

In light of the foregoing, the present invention provides a laminate sheet, a sanitary material, a medical material, and a method for manufacturing a laminate sheet that can improve the stretchability and the adhesion of the spunbond nonwoven fabric to the water-vapor permeable polyurethane film, and can reduce the tackiness properties.

MEANS FOR SOLVING THE PROBLEM

The present invention [1] includes a laminate sheet comprising: a water-vapor permeable polyurethane film having a hard segment phase; and a first spunbond nonwoven fabric disposed at one side in a thickness direction of the water-vapor permeable polyurethane film, the first spunbond nonwoven fabric containing stretchable fibers containing a thermoplastic polyurethane and non-stretchable fibers containing a polyolefin, wherein the hard segment phase has a melting point of 65°C or more and 140°C or less.

The present invention [2] includes the laminate sheet described in [1], wherein the water-vapor permeable polyurethane film has a soft segment phase, and the soft segment phase is formed by a reaction of a polyether polyol and a polyisocyanate.

The present invention [3] includes the laminate sheet described in [2], wherein the polyether polyol has a number average molecular weight of 1200 g/mol or more and 2800 g/mol or less.

The present invention [4] includes the laminate sheet described in any one of the above-described [1] to [3], further comprising: a second spunbond nonwoven fabric disposed at the other side in the thickness direction of the water-vapor permeable polyurethane film, the second spunbond nonwoven fabric containing stretchable fibers containing a thermoplastic polyurethane and non-stretchable fibers containing a polyolefin.

The present invention [5] includes the laminate sheet described in any one of the above-described [1] to [3], wherein the first spunbond nonwoven fabric is in direct contact with a one-side surface in the thickness direction of the water-vapor permeable polyurethane film.

The present invention [6] includes a sanitary material comprising the laminate sheet described in [1].

The present invention [7] includes a medical material comprising the laminate sheet described in [1].

The present invention [8] includes a method for manufacturing a laminate sheet, the method comprising: a step of mixing and joining stretchable fibers containing a thermoplastic polyurethane and non-stretchable fibers containing a polyolefin by heat fusion to prepare a spunbond nonwoven fabric; a step of preparing a water-vapor permeable polyurethane film having a hard segment phase, the hard segment phase having a melting point of 65°C or more and 140°C or less; a step of applying pressure on the spunbond nonwoven fabric against the water-vapor permeable polyurethane film to laminate the spunbond nonwoven fabric and the water-vapor permeable polyurethane film; and a step of subjecting the spunbond nonwoven fabric to a stretching process before the laminating step; and/or a step of subjecting the laminated spunbond nonwoven fabric and water-vapor permeable polyurethane film to a stretching process after the laminating step.

The present invention [9] includes the method described in [8], wherein the spunbond nonwoven fabric is in direct contact with a one-side surface in a thickness direction of the water-vapor permeable polyurethane film in the step of laminating the spunbond nonwoven fabric and the water-vapor permeable polyurethane film.

EFFECTS OF THE INVENTION

In the laminate sheet of the present invention, the melting point of the hard segment phase of the water-vapor permeable polyurethane film is the above-described upper limit or less, and the first spunbond nonwoven fabric includes the stretchable fibers containing a thermoplastic polyurethane. Thus, the stretchability of the laminate sheet can be improved while the adhesion of the first spunbond nonwoven fabric to the water-vapor permeable polyurethane film can be improved. Further, the melting point of the hard segment phase of the water-vapor permeable polyurethane film is the above-described lower limit or more, and the first spunbond nonwoven fabric includes the non-stretchable fibers containing a polyolefin. Thus, the tackiness properties of the laminate sheet can be reduced.

The method of manufacturing a laminate sheet of the present invention includes the step of subjecting the spunbond nonwoven fabric to a stretching process before the laminating of the spunbond nonwoven fabric and the water-vapor permeable polyurethane film and/or the step of subjecting the spunbond nonwoven fabric and water-vapor permeable polyurethane film to a stretching process after the laminating step, and thus can smoothly manufacture laminate sheets having the above-described properties.

The sanitary material and medical material of the present invention include the above-described laminate sheet, and thus can ensure excellent stretchability and excellent adhesion while simultaneously reducing the tackiness properties.

BRIEF DESCRIPTION OF THE DRAWINGS

FIG. 1 is a schematic view of one embodiment of the laminate sheet of the present invention.

FIG. 2 is a schematic view of a melt extrusion laminator that can implement the method for manufacturing a laminate sheet of the present invention.

DESCRIPTION OF THE EMBODIMENTS

<Laminate Sheet>

With reference to FIG. 1 and FIG. 2, a laminate sheet 1 is described as one embodiment of the laminate sheet of the present invention.

As illustrated in FIG. 1, the laminate sheet 1 includes a water-vapor permeable polyurethane film 2, a first spunbond nonwoven fabric 3, and a second spunbond nonwoven fabric 4.

The water-vapor permeable polyurethane film 2 has a film shape (flat board shape). Specifically, the water-vapor permeable polyurethane film 2 has a predetermined thickness, extends in a predetermined direction orthogonal to the thickness direction, and has a flat front surface and a flat back surface.

The thickness of the water-vapor permeable polyurethane film 2 is, for example, 1 μm or more, preferably 5 μm or more, more preferably 10 μm or more, and, for example, 50 μm or less, preferably 30 μm or less, more preferably 25 μm or less.

The water-vapor permeable polyurethane film 2 contains a water-vapor permeable polyurethane resin, and preferably consists of a water-vapor permeable polyurethane resin. The water-vapor permeable polyurethane film 2 has a hard segment phase and a soft segment phase.

The hard segment phase is a region (domain) made of a polyisocyanate and a low molecular-weight diol that is a chain extender, and is formed by a reaction of the polyisocyanate and the low molecular-weight diol.

The polyisocyanate contains, for example, diphenylmethane diisocyanate (MDI).

Examples of the diphenylmethane diisocyanate include 4,4'-diphenylmethane diisocyanate, 2,4'-diphenylmethane diisocyanate, and 2,2'-diphenylmethane diisocyanate.

The diphenylmethane diisocyanates can be used singly or in combination of two or more.

Among the diphenylmethane diisocyanates, 4,4'-diphenylmethane diisocyanate is preferable.

The content ratio of the diphenylmethane diisocyanate in the polyisocyanate is, for example, 80% by mass or more, preferably 90% by mass or more, and, for example, 100% by mass or less.

In addition to the diphenylmethane diisocyanate, the polyisocyanate can contain another polyisocyanate in a range that is not excluded from the scope of the present invention.

Examples of the other polyisocyanate include aliphatic polyisocyanate (for example, 1,5-pentamethylene diisocyanate (PDI) and 1,6-hexamethylene diisocyanate (HDI)), alicyclic polyisocyanate (for example, 3-isocyanatomethyl-3,5,5-trimethyl cyclohexylisocyanate (IPDI) and 1,3- and/or 1,4-bis (isocyanatomethyl)cyclohexane (HeXDI) (Trans-isomer, Cis-isomer, or a mixture thereof), methylenebis (cyclohexyl isocyanate), (4,4'-, 2,4'- or 2,2'-methylenebis (cyclohexyl isocyanate, Trans, Trans-isomer, Trans,Cis-isomer, or Cis,Cis-isomer of these, or a mixture thereof)) ($H_{12}$MDI)), araliphatic polyisocyanate (for example, m- and/or p-xylylene diisocyanate (XDI)), aromatic polyisocyanate other than diphenylmethane diisocyanate (for example, and tolylene diisocyanate (TDI)), a modified product modified from the polyisocyanates (for example, a multimer, an isocyanurate-modified product, a biuret-modified product, an allophanate-modified product, and a triol adduct), a modified product modified from diphenylmethane diisocyanate (for example, a multimer, a carbodiimide-modified product, and a polyphenylmethanepolyisocyanate (polymeric MDI)).

The content ratio of the other polyisocyanate in the polyisocyanate is, for example, 0% by mass or more, and, for example, 40% by mass or less, preferably 20% by mass or less, more preferably 10% by mass or less. The polyisocyanate more preferably consists of a diphenylmethane diisocyanate.

The low molecular-weight diol is, for example, a compound having a straight-chain alkylene group and two hydroxyl groups bonding to the alkylene group. The low molecular-weight diol has a number average molecular weight of 60 g/mol or more and 200 g/mol or less. Examples of the low molecular-weight diol include dihydric alcohols having 2 to 4 carbon atoms. Specific examples of the low molecular-weight diol include straight-chain alkane diols (for example, ethylene glycol, 1,3-propanediol, and 1,4-butanediol), and branched alkane diols (for example, 1,2-propanediol and 1,3-butanediol).

The low molecular-weight diols can be used singly or in combination of two or more.

The low molecular-weight diol preferably includes a straight-chain alkane diol having 2 to 4 carbon atoms, and more preferably includes 1,4-butanediol.

The hard segment phase has a melting point of 65°C or more, preferably 75°C or more, more preferably 80°C or more, and, 140°C or less, preferably 138°C or less, more preferably 135°C or less. The melting point of the hard segment phase can be measured by the method described below in Examples (the same applies hereinafter).

The concentration of the hard segment phase in the water-vapor permeable polyurethane film 2 is, for example, 5% by mass or more, preferably 10% by mass or more, more preferably 12% by mass or more, even more preferably 18% by mass or more, and, for example, 40% by mass or less, preferably 30% by mass or less.

The concentration of the hard segment phase is calculated from the following formula (1).

[MATH. 1]

$$\text{Hard segment concentration} = \frac{\text{CE (g)} + \dfrac{\text{CE (g)}}{\text{Mn of CE (g/mol)}} \times \text{M.W. of NCO (g/mol)}}{\text{High molecular-weight polyol (g)} + \text{NCO (g)} + \text{CE (g)}} \quad \cdots(1)$$

[in the formula (1), CE represents the low molecular-weight diol (chain extender), NCO represents the polyisocyanate, Mn represents the number average molecular weight, and M.W. represents the molecular weight]

The soft segment phase is a region (domain) formed of a high molecular-weight polyol and is formed by the reaction of the above-described polyisocyanate and the high molecular-weight polyol.

The high molecular-weight polyol is a compound having 2 or more hydroxyl groups and a number average molecular weight of 400 g/mol or more, preferably 500 g/mol or more, and, for example, 10000 g/mol or less, preferably 5000 g/mol or less. Examples of the high molecular-weight polyol include polyether polyols, polyester polyols, polycarbonate polyols, polyurethane polyols, epoxy polyols, vegetable oil polyols, polyolefin polyols, acrylic polyols, silicone polyols, fluorine polyols, and vinyl monomer-modified polyols.

Among the high molecular-weight polyols, a polyether polyol, a polyester polyol, and a polycarbonate polyol are preferable, and a polyether polyol is more preferable.

Examples of the polyether polyol include polyoxy (C2 to 3) alkylene polyols, polytetramethylene ether glycols, and polytrimethylene ether glycols.

Examples of the polyoxy (C2 to 3) alkylene polyol include an addition polymer of C2 to 3 alkylene oxides, for example, ethylene oxide and propylene oxide (including a random and/or block copolymer of 2 or more alkylene oxides) using a low molecular-weight polyol as an initiator.

The low molecular-weight polyol is a compound having 2 or more hydroxyl groups and a number average molecular weight of 60 g/mol or more and less than 400 g/mol, preferably 200 g/mol or less. Examples of the low molecular-weight polyol include the above-described low molecular-weight diols having a number average molecular weight of 60 g/mol or more and 200 g/mol or less, dihydric alcohols with a number average molecular weight of more than 100 g/mol and less than 400 g/mol (for example, alkane diols having 5 to 8 carbon atoms such as 1,5-pentanediol, 1,6-hexanediol, neopentyl glycol, and 3-methyl-1,5-pentanediol, and ether diols having 4 to 8 carbon atoms such as diethylene glycol and dipropylene glycol), trihydric alcohols (such as glycerin and trimethylolpropane), and tetrahydric alcohols (such as tetramethylolmethane (pentaerythritol) and diglycerin).

The low molecular-weight polyols as an initiator can be used singly or in combination of two or more.

Among the low molecular-weight polyols used as an initiator, a dihydric alcohol with a number average molecular weight of more than 100 g/mol and less than 400 g/mol is preferable, an ether diol having 4 to 8 carbon atoms is more preferable, and a dipropylene glycol is even more preferable.

Specific examples of the polyoxy (C2 to 3) alkylene polyol include polyoxyethylene glycol, polyoxypropylene glycol, and copolymerized polyols thereof. The polyoxy (C2 to 3) alkylene polyol can be produced, for example, in the presence of a known alkali catalyst or a phosphazenium catalyst.

Examples of the polytetramethylene ether glycol include a ring-opening polymerized product obtained by cation polymerization of tetrahydrofuran ((crystalline) polytetramethylene ether glycol), and noncrystalline polytetramethylene ether glycol obtained by copolymerizing a polymerization unit of tetrahydrofuran and the above-described low molecular-weight polyol or alkylene oxide.

Examples of the polytetramethylene ether glycol further include plants derived polytetramethylene ether glycol that is produced using tetrahydrofuran produced based on vegetable material such as furfural as a starting material.

Examples of the polytrimethylene ether glycol include a polyol produced by polycondensation reaction of plants derived 1,3-propanediol.

The high molecular-weight polyols can be used singly or in combination of two or more.

The high molecular-weight polyol preferably includes a polyether polyol, and more preferably consists of a polyether polyol. In other words, the soft segment phase is preferably formed by the reaction of the polyether polyol and polyisocyanate.

When the soft segment phase is formed by the reaction of the polyether polyol and polyisocyanate, the stretchability of the laminate sheet 1 can surely be improved while the adhesion of the first spunbond nonwoven fabric 3 to the water-vapor permeable polyurethane film 2 can surely be improved. Further, the water-vapor permeability of the laminate sheet 1 can be improved.

The polyether polyol preferably includes a polyoxy (C2 to 3) alkylene polyol and/or polytetramethylene ether glycol, more preferably consists of a polyoxy (C2 to 3) alkylene polyol and/or polytetramethylene ether glycol, and even more preferably consists of a polyoxy (C2 to 3) alkylene polyol and polytetramethylene ether glycol.

When the polyether polyol includes a polyoxy (C2 to 3) alkylene polyol and polytetramethylene ether glycol, the content ratio of the polyoxy (C2 to 3) alkylene polyol to 100% by mass of the total of the polyoxy (C2 to 3) alkylene polyol and polytetramethylene ether glycol is, for example, 60% by mass or more, preferably 70% by mass or more, more preferably 78% by mass or more, and, for example, 95% by mass or less, preferably 90% by mass or less.

The polyoxy (C2 to 3) alkylene polyol preferably includes an oxyethylene group. The content ratio of the oxyethylene group in the polyoxy (C2 to 3) alkylene polyol is, for example, 50% by mass or more, preferably 80% by mass or more, more preferably 85% by mass or more, and, for example, 100% by mass or less, preferably 95% by mass or less. The polyoxy (C2 to 3) alkylene polyol even more preferably consists of a polyoxyethylene polyol and/or a polyoxy (ethylene-propylene) copolymerized polyol.

The polyether polyol has a number average molecular weight of, for example, 400 g/mol or more, preferably 500 g/mol or more, more preferably 1200 g/mol or more, particularly preferably 1300 g/mol or more, especially preferably 1500 g/mol or more, and, for example, 4000 g/mol or less, preferably 3000 g/mol or less, more preferably 2800 g/mol or less, particularly preferably 2500 g/mol or less, especially preferably 2400 g/mol or less.

When the number average molecular weight of the polyether polyol is within the above-described range, the stretchability of the laminate sheet 1 can surely be improved while the adhesion of the first spunbond nonwoven fabric 3 to the water-vapor permeable polyurethane film 2 can surely be improved. When the number average molecular weight of the polyether polyol is the above-described lower limit or more, the tackiness properties of the laminate sheet 1 can surely be reduced.

The average number of the functional groups of the high molecular-weight polyol is, for example, 2.0 or more, and, for example, 4.0 or less, preferably 3.0 or less. The average number of the functional groups of the polyol can be calculated from the charging components.

The average values of the hydroxyl groups of the high molecular-weight polyol is, for example, 35 mgKOH/g or more, preferably 40 mgKOH/g or more, more preferably 45 mgKOH/g or more, and, for example, 115 mgKOH/g or less, preferably 87 mgKOH/g or less, more preferably 78 mgKOH/g or less.

Next, the production of the water-vapor permeable polyurethane film 2 is described.

To produce the water-vapor permeable polyurethane film 2, first, a water-vapor permeable polyurethane is prepared.

The water-vapor permeable polyurethane can be prepared by, for example, a prepolymer method.

In more detail, the above-described polyisocyanate and the above-described high molecular-weight polyol are reacted in the following ratio to synthesize an isocyanate group-terminated urethane prepolymer having an isocyanate group at its terminal.

At the time, the ratio (NCO/OH) of the isocyanate groups of the polyisocyanate to the hydroxyl groups of the high molecular-weight polyol is, for example, 1.9 or more, preferably 2.3 or more, more preferably 2.5 or more, and, for example, 3.5 or less, preferably 3.3 or less.

Next, the isocyanate group-terminated urethane prepolymer and the above-described low molecular-weight diol are reacted in a ratio in which the hard segment phase concentration is within the above-described range.

At the time, the ratio (NCO/OH) of the isocyanate groups of the isocyanate group-terminated urethane prepolymer to the hydroxyl groups of the low molecular-weight diol is, for example, 0.990 or more, preferably 1.005 or more, and, for example, 1.150 or less, preferably 1.100 or less.

In this manner, the water-vapor permeable polyurethane is prepared.

The water-vapor permeable polyurethane can be produced also by a one-shot method. In the one-shot method, for example, the polyisocyanate, high molecular-weight polyol, and low molecular-weight diol are reacted at a time in a ratio in which the hard segment phase concentration is within the above-described range to prepare the water-vapor permeable polyurethane.

The water-vapor permeable polyurethane produced as described above is a thermoplastic polyurethane. Thus, the produced water-vapor permeable polyurethane is crushed into pellets and then formed into a film or a sheet by, for example, a known method such as extrusion. The pelletization and extrusion step preferably includes a filtration operation, for example, described in JP patent No. 4332627 to reduce the fisheyes in the water-vapor permeable polyurethane.

As described above, the water-vapor permeable polyurethane film 2 is produced.

In the preparation of the water-vapor permeable polyurethane film 2, as necessary, a known additive such as an antioxidant (for example, a hindered phenolic antioxidant), an ultraviolet absorber (for example, a benzotriazole type ultraviolet absorber, a triazine type ultraviolet absorber, or a benzophenone type ultraviolet absorber), an lubricant, a release agent, or a pigment such as titanium oxide can be added at an appropriate time and ratio. In this case, the water-vapor permeable polyurethane film 2 contains a known additive in addition to the water-vapor permeable polyurethane.

The first spunbond nonwoven fabric 3 is disposed on one side in the thickness direction of the water-vapor permeable polyurethane film 2. More specifically, the first spunbond nonwoven fabric 3 is disposed on a surface of the water-vapor permeable polyurethane film 2 (one surface in the thickness direction) and is in direct contact with the surface of the water-vapor permeable polyurethane film 2.

The first spunbond nonwoven fabric 3 is laminated on the water-vapor permeable polyurethane film 2 without an adhesive. At least a part of the first spunbond nonwoven fabric 3 is fusion joined to the surface of the water-vapor permeable polyurethane film 2. Especially, at least a part of stretchable fibers containing a thermoplastic polyurethane described below is fusion joined to the surface of the water-vapor permeable polyurethane film 2.

The first spunbond nonwoven fabric 3 includes the stretchable fibers containing a thermoplastic polyurethane and non-stretchable fibers containing a polyolefin.

The stretchable fibers are polyurethane fibers made of a thermoplastic polyurethane as a material. Examples of the thermoplastic polyurethane include a thermoplastic polyurethane elastomer (A1) described in Japanese Unexamined Patent Publication No. 2008-213284.

The thermoplastic polyurethane is produced, for example, by the reaction of the above-described polyisocyanate (preferably, a diphenylmethane diisocyanate), the above-described high molecular-weight polyol (preferably, a polyester polyol), and the above-described low molecular-weight polyol (preferably, the above-described low molecular-weight diol). For example, the thermoplastic polyurethane can be produced by a known method such as a prepolymer method or a prepolymer method, similarly to the water-vapor permeable polyurethane.

In this manner, the thermoplastic polyurethane is produced.

In the preparation of the thermoplastic polyurethane, as necessary, the above-described known additive can be added at an appropriate time and ratio. In this case, the stretchable fibers contain the known additive in addition to the thermoplastic polyurethane.

The thermoplastic polyurethane has a hardness of, for example, 70 A or more, preferably 73 A or more, more preferably 75 A or more, and, for example, 95 A or less, preferably 90 A or less, more preferably 88 A or less, even more preferably 85 A or less. The hardness of the thermoplastic polyurethane can be measured at 23°C and 50% relative humidity by the method according to JIS K-7311 (durometer: type A) (the same applies hereinafter).

The thermoplastic polyurethane has a tensile strength of, for example, 20 MPa or more, preferably 30 MPa or more, and, for example, 70 MPa or less, preferably 60 MPa or less. The tensile strength of the thermoplastic polyurethane can be measured by the method according to JIS K-7311 (the same applies hereinafter).

The elongation at break of the thermoplastic polyurethane is, for example, 300% or more, preferably 400% or more, for example, 700% or less, preferably 600% or less. The elongation at break of the thermoplastic polyurethane can be measured by the method according to JIS K-7311 (the same applies hereinafter).

The content ratio of the stretchable fibers in the first spunbond nonwoven fabric 3 is, for example, 30% by mass or more, and, for further improvement of the stretchability, preferably 35% by mass or more. Similarly, for further improvement of the tackiness properties, the content ratio of the stretchable fibers is, for example, 70% by mass or less, preferably 60% by mass or less.

The non-stretchable fibers are polyolefin fibers made of a polyolefin as a material. Examples of the polyolefin include the polyolefin (B) described in Japanese Unexamined Patent Publication No. 2008-213284.

Specific examples of the polyolefin include polyethylenes, polypropylenes, and polyethylenepolypropylene copolymers.

The polyolefins can be used singly or in combination of two or more.

The polyolefin preferably includes polyethylene and/or polypropylene, more preferably includes polyethylene (more specifically, a high-density polyethylene) and polypropylene, and even more preferably consists of polyethylene (more specifically, a high-density polyethylene) and polypropylene.

The content ratio of the polypropylene to 100% by mass of the total of the polyethylene and polypropylene is, for example, 80% by mass or more, preferably 90% by mass or more, and, for example, 100% by mass or less, preferably 99% by mass or less.

The melt flow rate (MFR) of the polypropylene measured at a temperature of 230°C and a load of 2.16 kgf in conformity to ASTM D1238 is, for example, 10 g/10 minutes or more, preferably 20 g/10 minutes or more, and, for example, 1000 g/10 minutes or less, preferably 100 g/10 minutes or less.

The density of the polypropylene is, for example, 0.900 g/cm$^3$ or more and 0.920 g/cm$^3$ or less.

The polypropylene has a melting point of, for example, 120°C or more, preferably 140°C or more, and, for example, 200°C or less, preferably 180°C or less.

The melt flow rate (MFR) of the polyethylene measured at a temperature of 230°C and a load of 2.16 kgf in conformity to ASTM D1238 is, for example, 1 g/10 minutes or more, and, for further improvement of the spinnability, preferably 2 g/10 minutes or more. Similarly, the MFR of the polyethylene is, for example, 1000 g/10 minutes or less, and, for further improvement of the expansibility, preferably 100 g/10 minutes or less, more preferably 20 g/10 minutes or less.

The polyethylene has a density of, for example, 0.940 g/cm$^3$ or more, and, for further improvement of the spinnability, preferably 0.950 g/cm$^3$ or more. The density of the polyethylene is, for example, 0.980 g/cm$^3$ or less, and, for further improvement of the formability, preferably 0.975 g/cm$^3$ or less.

The polyethylene has a melting point of, for example, 100°C or more, preferably 120°C or more, and, for example, 180°C or less, preferably 160°C or less.

The content ratio of the non-stretchable fibers in the first spunbond nonwoven fabric 3 is, for example, 30% by mass or more, preferably 40% by mass or more, and, for example, 70% by mass or less, preferably 65% by mass or less.

The first spunbond nonwoven fabric 3 can contain other fibers in addition to the above-described stretchable fibers (polyurethane fibers) and non-stretchable fibers (polyolefin fibers) in a range that does not reduce the effects of the present invention. Examples of the other fibers include polystyrene fibers, polyvinyl chloride fibers, polyester fibers, and polyamide fibers.

The ratio of the other fibers in the first spunbond nonwoven fabric 3 is, for example, 0% by mass or more, and, for example, 10% by mass or less, preferably 5% by mass or less. The first spunbond nonwoven fabric 3 more preferably consists of stretchable fibers (polyurethane fibers) and non-stretchable fibers (polyolefin fibers).

The unit weight of the first spunbond nonwoven fabric 3 is, for example, 10 g/m$^2$ or more, preferably 15 g/m$^2$ or more, more preferably 20 g/m$^2$ or more, and, for example, 100 g/m$^2$ or less, preferably 40 g/m$^2$ or less, more preferably 30 g/m$^2$ or less.

Next, the production of the first spunbond nonwoven fabric 3 is described. The first spunbond nonwoven fabric 3 can be produced by, for example, the method of producing a stretchable nonwoven fabric described in Japanese Unexamined Patent Publication No. 2004-244791.

More specifically, the above-described thermoplastic polyurethane and the above-described polyolefin are individually fusion melt by a known extruder, and melt spinning is carried out by a spunbond method using the spunbond molding machine including the spinneret plate shown in FIG. 2 of Japanese Unexamined Patent Publication No. 2004-244791. In detail, the spinneret plate includes a plurality of first nozzles discharging the thermoplastic polyurethane and a plurality of second nozzles discharging the polyolefin. The stretchable fibers containing the thermoplastic polyurethane are discharged from the first nozzles and simultaneously the non-stretchable fibers containing the polyolefin are discharged from the second nozzles. In this manner, a web (mixed fibers) that is a mixture of the thermoplastic polyurethane stretchable fibers and the polyolefin non-stretchable fibers is deposited on the collection surface. Thereafter, the web is joined by heat fusion, for example, with heat embossing rolls to partially join the stretchable fibers and the non-stretchable fibers by heat fusion. In this manner, the first spunbond nonwoven fabric 3 is prepared.

The second spunbond nonwoven fabric 4 is disposed at the other side in the thickness direction of the water-vapor permeable polyurethane film 2. More specifically, the second spunbond nonwoven fabric 4 is disposed on a back surface of the water-vapor permeable polyurethane film 2 (one surface in the thickness direction), and is in direct contact with the back surface of the water-vapor permeable polyurethane film 2. The second spunbond nonwoven fabric 4 has the same structure as that of the first spunbond nonwoven fabric 3 and contains the stretchable fibers containing the thermoplastic polyurethane and the non-stretchable fibers containing the polyolefin. Thus, the description of the second spunbond nonwoven fabric 4 is omitted.

<Method for Manufacturing Laminate Sheet>

Next, with reference to FIG. 2, one embodiment of the method for manufacturing the laminate sheet 1 is described.

The method for manufacturing the laminate sheet 1 includes a step of preparing a spunbond nonwoven fabric, a step of preparing a water-vapor permeable polyurethane film, a step of laminating the spunbond nonwoven fabric and the water-vapor permeable polyurethane film, and a step of subjecting the laminated spunbond nonwoven fabric and water-vapor permeable polyurethane film to a stretching process.

The method for manufacturing the laminate sheet 1 is continuously carried out, for example, by a melt extrusion laminator 10 illustrated in FIG. 2.

The melt extrusion laminator 10 includes an extruder 11, a first lamination roll 12, a second lamination roll 13, a first delivery roll 14, a second delivery roll 15, a plurality of cooling rolls 16, and a stretching apparatus 17.

The extruder 11 is a known extruder and extrudes the above-described water-vapor permeable polyurethane into a sheet shape to discharge the water-vapor permeable polyurethane film 2. The water-vapor permeable polyurethane film 2 may not be solidified just after being discharged. The extruder 11 has a T die. The extruder 11 can adjust the temperature of the T die.

The first lamination roll 12 and the second lamination roll 13 are located on the downstream side in relation to the extruder 11 in a discharge direction. The first lamination roll 12 and the second lamination roll 13 face each other. Each of the first lamination roll 12 and the second lamination roll 13 is rotatable. The temperature of each of the first lamination roll 12 and the second lamination roll 13 is adjustable.

The water-vapor permeable polyurethane film 2 is discharged from the extruder 11 and fed between the first lamination roll 12 and the second lamination roll 13. The first lamination roll 12 and the second lamination roll 13 can laminate the first spunbond nonwoven fabric 3 and the second spunbond nonwoven fabric 4 on the fed water-vapor permeable polyurethane film 2 from both sides of the water-vapor permeable polyurethane film 2. The first lamination roll 12 is movable in relation to the second lamination roll 13. This motion enables the first lamination roll 12 to adjust a nip pressure applied on the first spunbond nonwoven fabric 3, water-vapor permeable polyurethane film 2, and second spunbond nonwoven fabric 4 that are held between the first lamination roll 12 and the second lamination roll 13.

The elongated first spunbond nonwoven fabric 3 is wound around the first delivery roll 14 in advance. The elongated first spunbond nonwoven fabric 3 is prepared by the above-described method in advance. The first spunbond nonwoven fabric 3 wound around the first delivery roll 14 is yet to be subjected to the stretching process. The first delivery roll 14 is rotatable and capable of delivering the first spunbond nonwoven fabric 3 yet to be stretched. From the upstream in the discharge direction in which the extruder 11 discharges the film, the first delivery roll 14 feeds the first spunbond nonwoven fabric 3 between the first lamination roll 12 and the second lamination roll 13 to bring the first spunbond nonwoven fabric 3 into contact with the one surface in the thickness direction of the water-vapor permeable polyurethane film 2.

The second delivery roll 15 is located at the opposite side to the first delivery roll 14 across the extruder 11. The elongated second spunbond nonwoven fabric 4 is wound around the second delivery roll 15 in advance. The elongated second spunbond nonwoven fabric 4 is prepared by the above-described method in advance. The second spunbond nonwoven fabric 4 wound around the second delivery roll 15 is yet to be subjected to the stretching process. The second delivery roll 15 is rotatable and capable of delivering the second spunbond nonwoven fabric 4 yet to be stretched. From the upstream in the discharge direction of the extruder 11, the second delivery roll 15 feeds the second spunbond nonwoven fabric 4 between the first lamination roll 12 and the second lamination roll 13 to bring the second spunbond nonwoven fabric 4 into contact with the other surface in the thickness direction of the water-vapor permeable polyurethane film 2.

The cooling rolls 16 cool a laminate 20 that is obtained by laminating the first spunbond nonwoven fabric 3, water-vapor permeable polyurethane film 2, and second spunbond nonwoven fabric 4 between the first lamination roll 12 and the second lamination roll 13. The temperature of each of the cooling rolls 16 is adjustable.

The stretching apparatus 17 is a gear stretching apparatus, for example, described in Japanese Unexamined Patent Publication No. 2004-244791. The stretching apparatus 17 stretches the laminate 20 by gear stretching after the cooling by the cooling rolls 16, thereby preparing the laminate sheet 1. The stretching apparatus 17 includes a first gear roll 18 and a second gear roll 19.

Each of circumferential surfaces of the first gear roll 18 and the second gear roll 19 has a plurality of gear teeth extending in a circumferential direction. The first gear roll 18 and the second gear roll 19 face each other to be engaged with each other. Each of the first gear roll 18 and the second gear roll 19 is rotatable. The laminate 20 is cooled by the cooling rolls 16, and fed and held between the first gear roll 18 and the second gear roll 19 to be gear stretched.

To produce the laminate sheet 1 with the melt extrusion laminator 10, first, (pellets of) the water-vapor permeable polyurethane prepared as described above (are) is set in the extruder 11.

It is preferable to reduce the water content of the pellets of the water-vapor permeable polyurethane to, for example, 500 ppm or less, preferably 300 ppm or less, more preferably 200 ppm or less by using a dehumidification dryer in order to improve the mechanical properties and water-vapor permeability of the water-vapor permeable polyurethane film. It is preferable to connect the dehumidification dryer to the extruder to convey the pellets without contact with air.

The temperature of the T die of the extruder 11 is adjusted to, for example, 180°C or more and 250°C or less. Thereafter, the molten water-vapor permeable polyurethane is extruded into a film shape from the T die. In this manner, the water-vapor permeable polyurethane film 2, which is made of the molten water-vapor permeable polyurethane, is prepared.

Then, the molten water-vapor permeable polyurethane film 2 is fed between the first lamination roll 12 and the second lamination roll 13. At the time, the first spunbond nonwoven fabric 3 is delivered from the first delivery roll 14 and fed between the molten water-vapor permeable polyurethane film 2 and the first lamination roll 12 while the second spunbond nonwoven fabric 4 is delivered from the second delivery roll 15 and fed between the molten water-vapor permeable polyurethane film 2 and the second lamination roll 13.

In this manner, the first spunbond nonwoven fabric 3 is in direct contact with the surface of the molten water-vapor permeable polyurethane film 2 (the one surface in the thickness direction). The second spunbond nonwoven fabric 4 is in direct contact with the back surface of the molten water-vapor permeable polyurethane film 2 (the other surface in the thickness direction).

Then, the first spunbond nonwoven fabric 3, the molten water-vapor permeable polyurethane film 2, and the second spunbond nonwoven fabric 4 are held between the first lamination roll 12 and the second lamination roll 13. Thus, the nip pressure of the first lamination roll 12 and the second lamination roll 13 is exerted to move the first spunbond nonwoven fabric 3 and the second spunbond nonwoven fabric 4 onto the whole of the molten water-vapor permeable polyurethane film 2 in a width direction.

The nip pressure (gauge pressure) is, for example, 0.1 MPaG or more, preferably 0.3 MPaG or more, and, for example, 2.0 MPaG or less, preferably 1.0 MPaG or less, more preferably 0.6 MPaG or less.

The temperature of each of the first lamination roll 12 and the second lamination roll 13 is, for example, 10°C or more, preferably 15°C or more, more preferably 20°C or more, and, for example, 50°C or less, preferably 40°C or less.

Then, the first spunbond nonwoven fabric 3 and the second spunbond nonwoven fabric 4 are laminated on both surfaces of the molten water-vapor permeable polyurethane film 2.

In this manner, the first spunbond nonwoven fabric 3, the water-vapor permeable polyurethane film 2, and the second spunbond nonwoven fabric 4 are sequentially laminated, thereby preparing the laminate 20.

In the laminate 20, the molten water-vapor permeable polyurethane located on the surface of the water-vapor permeable polyurethane film 2 is fusion joined to at least a part (especially, the stretchable fibers) of the first spunbond nonwoven fabric 3. Further, the molten water-vapor permeable polyurethane located on the back surface of the water-vapor permeable polyurethane film 2 is fusion joined to at least a part (especially, the stretchable fibers) of the second spunbond nonwoven fabric 4.

Thereafter, the laminate 20 passes on the second lamination roll 13 and is drawn through the cooling rolls 16.

In this manner, the molten water-vapor permeable polyurethane film 2 is solidified and the first spunbond nonwoven fabric 3 and the second spunbond nonwoven fabric 4 are adhered to the water-vapor permeable polyurethane film 2 without using an adhesive.

The cooling rolls 16 have, for example, a temperature lower than or equal to that of the second lamination roll 13. The cooling rolls 16 have a temperature of, for example, 0°C or more, preferably 10°C or more, and, for example, 40°C or less, preferably 30°C or less.

Thereafter, the cooled laminate 20 is fed and held between the first gear roll 18 and the second gear roll 19 to be stretched by the gear stretching process.

The stretching ratio of the gear stretching process is, for example, 1.5 times or more, preferably 2.0 times or more, and, for example, 5 times or less, preferably 4.0 times or less. When the stretching ratio is the above-described lower limit or more, the stretchability tends to further improve. When the stretching ratio is the above-described upper limit or less, the damage to the water-vapor permeable polyurethane film 2, and the first spunbond nonwoven fabric and second spunbond nonwoven fabric reduces. Thus, the wearing by fiber breakage, the reduction in strength, and the reduction in peeling strength tend to further be prevented.

The stretching direction of the gear stretching process may be a machine direction (MD) or a cross direction (CD). In view of the running stability during the molding process, the stretching direction is preferably the cross direction (CD). When the gear process is carried out in the cross direction (CD), a known continuous process or batch process may be used. When the stretching is carried out in the machine direction (MD), a known gear process method or roll stretching method may be used. Alternatively, depending on the purpose, the machine direction (MD) and cross direction (CD) can be combined.

The laminate sheet 1 is produced as described above.

Examples of the use of the laminate sheet 1 include domestic use, medical and hygienic use, industrial use, civil engineering and architectural use, agricultural and horticultural use, and clothing use. More specifically, the laminate sheet 1 is used for the sanitary materials (for example, disposable diapers, menstrual pads, urinal pads, gauzes, masks, wet wipes, laboratory coats, and dust masks), the medical materials (for example, surgical coats and medical bandages), and birthing pads, and medical caps. Preferably, the laminate sheet 1 is used for sanitary materials and medical materials. In other words, the sanitary materials include the above-described laminate sheet 1. The medical materials include the above-described laminate sheet 1. The sanitary materials and medical materials include the above-described laminate sheet 1 and thus can achieve excellent stretchability and excellent adhesion while simultaneously reducing the tackiness properties.

Further, the laminate sheet 1 can be used for housewares materials such as rugs, base fabrics for rugs, storage bags, wrapping cloths, garment covers, tea bags, drainage sheets, wipers, shoes, slippers, and bags. Further, the laminate sheet 1 can be used for industrial materials such as interior accessories for cars such as floor mats, various filters for cars, polishing materials, papermaking felts, wire banding tapes, battery cell separators, air filters, and liquid filters. Furthermore, the laminate sheet 1 can be used for interlinings for cloths, pads for brassieres, shoulder pads, event jumpers, and synthetic leathers (such as synthetic leather base fabrics and vinyl chloride leathers).

<Operations and Effects>

In the laminate sheet 1, the hard segment phase of the water-vapor permeable polyurethane film 2 has a melting point of the above-described upper limit or less, and the first spunbond nonwoven fabric 3 contains the stretchable fibers containing the thermoplastic polyurethane. Thus, the stretchability of the laminate sheet 1 can be improved while the adhesion of the first spunbond nonwoven fabric 3 to the water-vapor permeable polyurethane film 2 can be improved. Further, the hard segment phase of the water-vapor permeable polyurethane film 2 has a melting point of the above-described lower limit or more, and the first spunbond nonwoven fabric 3 contains the non-stretchable fibers containing the polyolefin. Thus, the tackiness properties of the laminate sheet 1 can be reduced.

Furthermore, the soft segment phase of the water-vapor permeable polyurethane film 2 is preferably formed by the reaction of the polyether polyol and polyisocyanate. Thus, the stretchability of the laminate sheet 1 can surely be improved while the adhesion of the first spunbond nonwoven fabric 3 to the water-vapor permeable polyurethane film 2 can surely be improved. In addition, the water-vapor permeability of the laminate sheet 1 can be improved.

The number average molecular weight of the polyether polyol forming the soft segment phase is preferably within the above-described range or more. Thus, the stretchability of the laminate sheet 1 can surely be improved while the adhesion of the first spunbond nonwoven fabric 3 to the water-vapor permeable polyurethane film 2 can surely be improved. In addition, the tackiness properties of the laminate sheet 1 can surely be reduced.

As illustrated in FIG. 2, in the method for manufacturing the laminate sheet 1, the first spunbond nonwoven fabric 3, the water-vapor permeable polyurethane film 2, and the second spunbond nonwoven fabric 4 are laminated, and thereafter the stretching process is carried out by the stretching apparatus 17. Thus, the laminate sheet 1 having the above-described properties can smoothly be manufactured.

<Variations>

The above-described laminate sheet 1 includes the water-vapor permeable polyurethane film 2, the first spunbond nonwoven fabric 3, and the second spunbond nonwoven fabric 4. However, the present invention is not limited to the above description.

The laminate sheet 1 may not include the second spunbond nonwoven fabric 4 and may consist of the water-vapor permeable polyurethane film 2 and the first spunbond nonwoven fabric 3.

Alternatively, the laminate sheet 1 can include another layer in addition to the water-vapor permeable polyurethane film 2 and the first spunbond nonwoven fabric 3 in a range that does not reduce the effects of the present invention. Examples of the other layer include spunbond nonwoven fabrics other than the above-described spunbond nonwoven fabrics, meltblown nonwoven fabrics, wet nonwoven fabrics, dry nonwoven fabrics, needle punched nonwoven fabrics, stitch bonded nonwoven fabrics, films, natural fiber assemblies (for example, silks, gauzes, cottons and rayons).

In the above-described method for manufacturing the laminate sheet 1, the laminated spunbond nonwoven fabric and water-vapor permeable polyurethane film are subjected to the stretching process after the laminating step. However, the spunbond nonwoven fabric may be subjected to the stretching process before the laminating step.

In other words, the laminate sheet 1 requires the stretching process of the first spunbond nonwoven fabric 3 and second spunbond nonwoven fabric 4. However, the stretching process may be carried out before and/or after the laminating step. The stretching process before the laminating step is preferable because it tends to further improve the peeling strength. A gear stretching process is preferred when the stretching process is carried out in the cross direction (CD) before the laminating step. A known method such as roll stretching or gear stretching can be used when the stretching process is carried out in the machine direction (MD).

The stretching ratio of the stretching process before the laminating step is, for example, 1.5 times or more, preferably 2.0 times or more, and, for example, 5 times or less, preferably 4.0 times or less. When the stretching ratio is the above-described lower limit or more, the stretchability tends to further improve. When the stretching ratio is the above-described upper limit or less, the damage to the water-vapor permeable polyurethane film 2, the first spunbond nonwoven fabric, and the second spunbond nonwoven fabric reduces. Thus, the wearing by fibers breakage, the reduction in strength, and the reduction in peeling strength tend to further be prevented.

The stretching process before the laminating step may be carried out in the machine direction (MD) or in the cross direction (CD). In view of the running stability of the spunbond nonwoven fabric during the laminating step, the stretching process before the laminating step is carried out preferably in the cross direction (CD).

## Example

The present invention is more specifically described below with reference to Examples. The present invention is not limited to Examples in any way. The specific numeral values used in the description below, such as mixing ratios (contents), physical property values, and parameters can be replaced with the corresponding mixing ratios (contents), physical property values, and parameters in the above-described "DESCRIPTION OF THE EMBODIMENT", including the upper limit values (numeral values defined with "or less", and "less than") or the lower limit values (numeral values defined with "or more", and "more than"). The "parts" and "%" are based on mass unless otherwise specified.

### <Preparation of High Molecular-weight Polyol>

#### <<Preparation Example 1: Polyol A»

An alcoholate of a dipropylene glycol and a phosphazenium compound (tetrakis[tris(dimethylamino)phosphoranylideneamino]phosphonium chloride) as a catalyst was prepared in the same method as Example 2 described in Japanese Unexamined Patent Publication No. H11-106500.

Next, ethylene oxide was subjected to addition polymerization in the presence of the alcoholate and thereafter the catalyst was removed, thereby preparing a polyol A (polyoxyethylene polyol). The polyol A had a number average molecular weight (Mn) of 1950. The polyol A had an average hydroxyl group value of 57.5 mgKOH/g.

#### <<Preparation Example 2: Polyol B>>

A polytetramethylene ether glycol (PTMEG, manufactured by Mitsui Chemicals, Inc.) was prepared as a polyol B. The polyol B had a number average molecular weight (Mn) of 995. The polyol B had an average hydroxyl group value of 112.8 mgKOH/g.

#### <<Preparation Example 3: Polyol C>>

An alcoholate of a dipropylene glycol and a potassium hydroxide as a catalyst was prepared by a common method.

Next, propylene oxide was subjected to addition polymerization in the presence of the alcoholate, the propylene oxide and ethylene oxide were copolymerized, and thereafter the catalyst was removed, thereby preparing a polyol C (polyoxyethylene polyoxypropylene polyol). The oxyethylene group content in the polyol C was 80% by mass, and the oxypropylene group content was 20% by mass. The polyol C had a number average molecular weight (Mn) of 2270. The polyol C had an average hydroxyl group value of 49.5 mgKOH/g.

<<Preparation Example 4: Polyol D>>

An alcoholate of a dipropylene glycol and a potassium hydroxide as a catalyst was prepared by a common method.

Next, propylene oxide was subjected to addition polymerization in the presence of the alcoholate, ethylene oxide was subjected to addition polymerization, and thereafter the catalyst was removed, thereby preparing a polyol D (polyoxyethylene polyoxypropylene polyol). The oxyethylene group content in the polyol D was 90% by mass, and the oxypropylene group content was 10% by mass. The polyol D had a number average molecular weight (Mn) of 2945. The polyol D had an average hydroxyl group value of 38.1 mgKOH/g.

<<Preparation Example 5: Polyol E>>

An alcoholate of a dipropylene glycol and a potassium hydroxide as a catalyst was prepared by a common method.

Next, propylene oxide was subjected to addition polymerization in the presence of the alcoholate, ethylene oxide was subjected to addition polymerization, and thereafter the catalyst was removed, thereby preparing a polyol E (polyoxyethylene polyoxypropylene polyol). The oxyethylene group content in the polyol E was 90% by mass, and the oxypropylene group content was 10% by mass. The polyol D had a number average molecular weight (Mn) of 997. The polyol E had an average hydroxyl group value of 112.5 mgKOH/g.

<<Preparation Example 6: Polyol F>>

A polycarbonate diol (ETERNACOLL UH- 200, manufactured by UBE Corporation) was prepared as a polyol F. The polyol F had a number average molecular weight (Mn) of 1993. The polyol F had an average hydroxyl group value of 56.3 mgKOH/g.

<<Preparation Example 7: Polyol G>>

A polycaprolactone diol (PLACCEL 220N, manufactured by Daicel Corporation) was prepared as a polyol G. The polyol G had a number average molecular weight (Mn) of 2008. The polyol G had an average hydroxyl group value of 55.9 mgKOH/g.

<Preparation of Water-vapor-permeable Polyurethane>

<<Preparation Example 1: Water-vapor permeable Polyurethane A>>

A reactor vessel equipped with an agitator was charged with a polyol, an antioxidant (trade name: ADK STAB AO-80, manufactured by ADEKA Corporation), and an ultraviolet absorber (a benzotriazole type ultraviolet absorber, trade name: JF-83, manufactured by JOHOKU CHEMICAL CO.,LTD) according to the formulation of Table 1, and the mixture was dissolved at 80°C for one hour.

Next, the reactor vessel was charged with 4,4'-diphenylmethane diisocyanate (trade name: COSMONATE MDI-PH, manufactured by Mitsui Chemicals and SKC Polyurethanes Inc.) according to the formulation of Table 1 and the mixture was reacted at 80°C for four hours, thereby preparing an isocyanate group-terminated urethane prepolymer (hereinafter, referred to as prepolymer).

Thereafter, the prepolymer was adjusted to 80°C. 1,4-butanediol dissolved at 80°C in advance (manufactured by Mitsui Chemicals, Inc. and hereinafter referred to as 1,4-BD) and ethylene bis stearic acid amide were added, and the mixture was sufficiently stirred and mixed while the mixing of air bubbles was prevented, thereby producing a reaction mixture.

Next, a vessel processed with fluorine and adjusted to 160°C in advance was quickly charged with the reaction mixture and the mixture was reacted at 160°C for 2.5 hours. Thereafter, the vessel was moved to another oven adjusted to 105°C to react the mixture at 105°C for 22 hours, thereby preparing a water-vapor permeable polyurethane A.

Thereafter, the water-vapor permeable polyurethane A was annealed to a room temperature (25°C) and crushed. Next, the water-vapor permeable polyurethane A was cured at a room temperature (25°C) for 2 weeks, and the crushed water-vapor permeable polyurethane A was dried at 80°C in a dehumidification dryer, and formed into pellets using a single-axis extruder. The temperature of the extruder was set to 180°C to 215°C from the hopper to the tip of the T die to prevent a foreign matter from being found in the strand.

The produced pellets were dried again at 80°C in the dehumidification dryer, and a water-vapor permeable polyurethane film A with a thickness of 20 μm was produced from the T die of the single-axis extruder. To prevent a foreign matter from being found in the water-vapor permeable polyurethane film A, the temperature of the cylinder was set to 180°C to 215°C, and the temperature of the connecting tube and the T die was set to 195°C to 218°C.

The water-vapor permeable polyurethane film A was placed on a sheet of release paper and cured in a constant temperature and humidity room at 23°C and 55% relative humidity for a week. Thereafter, the melting point of the hard segment phase was measured and was 104°C. Tables 1 and 2 show the formulation (charging amount) of each material of the water-vapor permeable polyurethane, the hard segment concentration of each of the water-vapor permeable polyurethane films, and the melting point of the hard segment phase of each of the water-vapor permeable polyurethane films.

<<Preparation Example 2: Water-vapor permeable Polyurethane B>>

Except that the following points were changed, a water-vapor permeable polyurethane B and a water-vapor permeable polyurethane film B were prepared in the same manner as Preparation Example 1.

- The formulation of each material was changed according to Table 1.
- The drying temperature of the dehumidification dryer was changed to 70°C.
- The set temperature of the extruder was changed to 170°C to 210°C.
- The temperature of the cylinder was changed to 175°C to 210°C.
- The temperature of the connecting tube and the T die was changed to 185°C to 210°C.

<<Preparation Example 3: Water-vapor permeable Polyurethane C»

Except that the following points were changed, a water-vapor permeable polyurethane C and a water-vapor permeable polyurethane film C were prepared in the same manner as Preparation Example 1.

- The formulation of each material was changed according to Table 1.
- The reaction time in the preparation of the prepolymer was changed to 3.5 hours.

<<Preparation Example 4: Water-vapor permeable Polyurethane D>>

Except that the following points were changed, a water-vapor permeable polyurethane D and a water-vapor permeable polyurethane film D were prepared in the same manner as Preparation Example 1.

- The formulation of each material was changed according to Table 1.
- The drying temperature of the dehumidification dryer was changed to 70°C.
- The set temperature of the extruder was changed to 170°C to 210°C.
- The temperature of the cylinder was changed to 175°C to 210°C.
- The temperature of the connecting tube and the T die was changed to 185°C to 210°C.

<<Preparation Examples 5 to 8: Water-vapor permeable Polyurethanes E to H>>

Except that the formulation of each material was changed according to Table 1, water-vapor permeable polyurethanes E to H and water-vapor permeable polyurethane films E to H were prepared in the same manner as Preparation Example 1.

<<Preparation Example 9: Water-vapor permeable Polyurethane I>>

Except that the following points were changed, a water-vapor permeable polyurethane I and a water-vapor permeable polyurethane film I were prepared in the same manner as Preparation Example 1.

- The formulation of each material was changed according to Table 2.
- The set temperature of the extruder was changed to 195°C to 223°C.
- The temperature of the cylinder was changed to 190°C to 225°C.
- The temperature of the connecting tube and the T die was changed to 195°C to 223°C.

<<Preparation Example 10: Water-vapor permeable Polyurethane J>>

Except that the formulation of each material was changed according to Table 2, a water-vapor permeable polyurethane J was prepared in the same manner as Preparation Example 1. Thereafter, the water-vapor permeable polyurethane J was crushed. Next, the crushed water-vapor permeable polyurethane J was cured at a room temperature (25°C) for two weeks, and then dried at 80°C in a dehumidification dryer. Thereafter, the water-vapor permeable polyurethane J was formed into pellets with a single-axis extruder while the temperature of the extruder was adjusted to 195°C to 230°C.

However, the strand foamed without melting. This was presumably because the ratio ([NCO]/[OH]) of the hydroxyl group concentration of the 1,4-BD to the isocyanate group concentration of the prepolymer was 1.32 and the isocyanate group induced a side reaction caused by the chemical cross-link during the synthesis of the water-vapor permeable polyurethane. Thus, the formation of the water-vapor permeable polyurethane film J failed.

<<Preparation Example 11: Water-vapor permeable Polyurethane K>>

Except that the chain extender was changed to a neopentyl glycol and the formulation of each material was changed according to Table 2, a water-vapor permeable polyurethane K was prepared in the same manner as Preparation Example 1. Thereafter, the water-vapor permeable polyurethane K was cured at a room temperature (25°C) for two weeks, and the disappearance of the isocyanate group of the water-vapor permeable polyurethane was confirmed by an infrared spectroscopic measurement method.

However, the water-vapor permeable polyurethane K had a very high adhesion and this made it difficult to dehumidify and dry the water-vapor permeable polyurethane K and form the water-vapor permeable polyurethane K into a strand with the single-axis extruder. It is presumed that the chain extender was a neopentyl glycol and the ratio [NCO]/[OH] was approximately 0.66 and thus the hard segment phase was not agglutinated and the resin was not solidified. Thus, the formation of the water-vapor permeable polyurethane film K failed.

<<Preparation Example 12: Water-vapor permeable Polyurethane L>>

Except that the following points were changed, a water-vapor permeable polyurethane L and a water-vapor permeable polyurethane film L were prepared in the same manner as Preparation Example 1.

- The formulation of each material was changed according to Table 2.
- The drying temperature of the dehumidification dryer was changed to 70°C.
- The set temperature of the extruder was changed to 170°C to 210°C.
- The temperature of the cylinder was changed to 175°C to 210°C.
- The temperature of the connecting tube and the T die was changed to 185°C to 210°C.

<<Preparation Example 13: Water-vapor permeable Polyurethane M»

Except that the following points were changed, a water-vapor permeable polyurethane M and a water-vapor permeable polyurethane film M were prepared in the same manner as Preparation Example 1.

- The formulation of each material was changed according to Table 2.
- The reaction time in the preparation of the prepolymer was changed to 3 hours.
- The set temperature of the extruder was changed to 195°C to 225°C.
- The temperature of the cylinder was changed to 195°C to 228°C.
- The temperature of the connecting tube and the T die was changed to 195°C to 227°C.

<Melting Point (Unit: °C) of Hard Segment Phase of Water-vapor Permeable Polyurethane by Differential Scanning Calorimeter (DSC)>

A differential scanning calorimeter (trade name: EXSTAR 6000 PC station and DSC 220C, manufactured by SII NanoTechnology Inc.) was used to measure the melting point of the hard segment phase of the water-vapor permeable polyurethane of each Preparation Example.

Specifically, approximately 8 mg of the water-vapor permeable polyurethane film of each Preparation Example was sliced into a thin shape that can be in as tight contact with an aluminum pan as possible. The water-vapor permeable polyurethane film was crimped with a cover on the aluminum pan, thereby producing a measurement specimen (sample). Alumina was processed in the same manner, thereby producing a reference specimen. The sample specimen and reference specimen were set at a predetermined location in a cell and cooled to - 100°C at a speed of 10°C/min in a nitrogen stream at a flow rate of 40 NmL/min and held at - 100°C for 5 minutes, and then the temperature was raised to 270°C at a speed of 10°C/min. The temperature at the endothermic peak in the temperature rise was measured as the melting point of the hard segment phase of the water-vapor permeable polyurethane. The results are shown in Tables 1 and 2.

<Preparation of Thermoplastic Polyurethane>

<<Preparation Example 14: Thermoplastic Polyurethane>>

In a nitrogen atmosphere, a reactor vessel equipped with an agitator was charged with 245 parts by mass of a polyester polyol (hydroxyl group value of 112.1 mgKOH/g, trade name: Takelac U-2410, manufactured by Mitsui Chemicals Inc.), 490 parts by mass of a polyester polyol (hydroxyl group value of 56.2 mgKOH/g, trade name: Takelac U-2420, manufactured by Mitsui Chemicals Inc.), 1.97 parts by mass of an antioxidant (trade name: ADK STAB AO-80, manufactured by ADEKA Corporation), 0.8 parts by mass of bis (2,6-diisopropylphenyl)carbodiimide (trade name: stabilizer 7000, manufactured by RASCHIG GmbH), and 1.1 parts by mass of an ultraviolet absorber (benzotriazole type ultraviolet absorber, trade name: JF-83, manufactured by JOHOKU CHEMICAL CO.,LTD), and the mixture was dissolved at 80°C for one hour.

Next, the reactor vessel was charged with 202 parts by mass of 4,4'-diphenylmethane diisocyanate (trade name: COSMONATE MDI-PH, manufactured by Mitsui Chemicals and SKC Polyurethanes Inc.), and the mixture was reacted at 80°C for 3 hours, thereby preparing an isocyanate group-terminated urethane prepolymer (hereinafter, referred to as prepolymer).

Thereafter, 50.1 parts by mass of 1,4-butanediol (manufactured by Mitsui Chemicals, Inc.), 0.99 parts by mass of ethylene bis stearic acid amide, and 0.5 parts by mass of ethylene bis montanic acid amide were dissolved at 80°C in advance and added to the prepolymer adjusted to 80°C. The mixture was sufficiently stirred and mixed while the mixing of air bubbles was prevented, thereby producing a reaction mixture.

Next, a vessel processed with fluorine and adjusted to 170°C in advance was quickly charged with the reaction mixture and the mixture was reacted at 170°C for two hours. Thereafter, the vessel was moved to another oven adjusted to 110°C to react the mixture at 110°C for 20 hours, thereby preparing a thermoplastic polyurethane. Thereafter, the thermoplastic polyurethane was annealed to a room temperature (25°C) and crushed.

Next, the crushed thermoplastic polyurethane was cured at a room temperature (25°C) for two weeks, and dried at 80°C in a dehumidification dryer, and formed into pellets using a single-axis extruder. The temperature of the extruder was set to 180°C to 215°C from the hopper to the tip of the T die to prevent a foreign matter from being found in the strand.

The produced pellets were dried again at 80°C in a dehumidification dryer and injection molded. The injection-molded thermoplastic polyurethane (hereinafter, referred to as TPU) had a hardness of 81 A, a tensile strength of 54 MPa, and an elongation at break of 580%.

<Preparation of Spunbond Nonwoven Fabric>

<<Preparation Example 15: PO/TPU Spunbond Nonwoven Fabric>>

94 parts by mass of a propylene homopolymer (hereinafter, referred to as PP), and 6 parts by mass of a high-density polyethylene (hereinafter, referred to as HDPE) were mixed, thereby preparing a thermoplastic polyolefin (hereinafter, referred to as PO).

The PP had a MFR of 60 g/10 minutes (measured at a temperature of 230°C and a load of 2.16 kgf in conformity to ASTM D1238), a density of 0.91 g/cm$^3$, and a melting point of 160°C. The HDPE had a MFR of 5 g/10 minutes (measured at a temperature of 190°C and a load of 2.16 kgf in conformity to ASTM D1238), a density of 0.97 g/cm$^3$, and a melting point of 134°C.

The TPU prepared in Preparation Example 14 and the PO were separately molten with an extruder, and melt spinning was carried out by a spunbond method using a spunbond molding machine with a spinneret plate illustrated in FIG. 2 of Japanese Unexamined Patent Publication No. 2004-244791 under the conditions of the resin temperature of 210°C, the T die temperature of 210°C, the temperature of the cooling air of 20°C, the discharge amount per hole of 0.7 g/min/hole, and the rate of stretching air of 3500 m/min, thereby depositing a web of the mixture of stretchable fibers containing the TPU and non-stretchable fibers containing the PO on the collection surface.

The spinneret plate had the nozzle arrangement pattern illustrated in FIG. 2 of Japanese Unexamined Patent Publication No. 2004-244791, and discharged the web in a ratio of 40% by mass of the TPU stretchable fibers and 60% by mass of the PO non-stretchable fibers.

The web was produced at a web forming rate of 20 m/min, and next the produced web was processed at 110°C by a heat embossing process (molded shape: rhombus and area ratio of 18%), thereby preparing a PO/TPU spunbond nonwoven fabric with a unit weight of 28 g/m$^2$.

<<Preparation Example 16: PO Spunbond Nonwoven Fabric>>

Only the PP was molten with the extruder, and melt spinning was carried out by a spunbond method using a spunbond molding machine with spinnerets with a nozzle diameter of 0.6 mmφ under the same conditions as Preparation Example 15, thereby depositing a web of non-stretchable fibers (PP fibers) consisting of the PP on the collection surface.
The web was produced at a web forming rate of 20 m/min, and next the produced web was processed at 140°C by a heat embossing process (molded shape: rhombus and area ratio of 18%), thereby preparing a PO spunbond nonwoven fabric with a unit weight of 28 g/m$^2$.

<<Preparation Example 17: TPU Spunbond Nonwoven Fabric>>

Only the TPU was molten with the extruder, and melt spinning was carried out by a spunbond method using a spunbond molding machine with spinnerets with a nozzle diameter of 0.75 mmφ under the same conditions as Preparation Example 15, thereby depositing a web of non-stretchable fibers (TPU fibers) consisting of the TPU on the collection surface.
A polyester nonwoven fabric (Mitsui Chemicals' REEMAY R-250, a unit weight of 18 g/m$^2$) was placed on the collection surface in advance so that the polyester nonwoven fabric suppressed the stickiness of the TPU fibers to the collection surface by laminating the TPU fibers on the polyester nonwoven fabric.
The web consisting of the TPU fibers was laminated on the polyester nonwoven fabric, and thereafter the same polyester nonwoven fabric as described was further laminated on the web consisting of the TPU fibers. In this manner, the web consisting of the TPU fibers was held between the two polyester nonwoven fabrics.

<<Preparation Example 18>>

The PO/TPU spunbond nonwoven fabric produced in Preparation Example 15 was stretched by a batch stretching apparatus under the conditions of a tension rate of 300 mm/min and a stretching ratio in the CD direction of 3.0 times, and immediately recovered to the original length at the same rate, thereby preparing a stretched and recovered TPU spunbond nonwoven fabric.
The web was produced at a web forming rate of 20 m/min, and next the produced web was processed at 110°C by a heat embossing process (molded shape: rhombus and area ratio of 18%). Thereafter, the two polyester nonwoven fabrics were removed, thereby preparing a TPU spunbond nonwoven fabric with a unit weight of 28 g/m$^2$.

<Preparation of Laminate Sheet>

«Example 1»

The water-vapor permeable polyurethane A of Preparation Example 1 and the PO/TPU spunbond nonwoven fabric of Preparation Example 15 were set in the melt extrusion laminator illustrated in FIG. 2.
Next, the temperature of the T die at the tip of an extruder was set to 215°C to form the molten water-vapor permeable polyurethane into a film from the extruder. The molten water-vapor permeable polyurethane was fed between the first lamination roll and the second lamination roll. The first lamination roll and the second lamination roll were set to a temperature of 30°C and a nip pressure of 0.5 MPaG.
Next, pressure was applied on both surfaces of the molten water-vapor permeable polyurethane film so that the spunbond nonwoven fabrics are pressed against the water-vapor permeable polyurethane film, thereby laminating the two spunbond nonwoven fabrics on the water-vapor permeable polyurethane film. Each cylinder of the extruder was set to the same temperature as Preparation Example 1. At the time, the thickness of the water-vapor permeable polyurethane film was maintained at 20 μm by adjusting the number of revolutions of the extruder.
Thereafter, as illustrated in FIG. 1, the water-vapor permeable polyurethane film laminated by the two spunbond nonwoven fabrics was passed and cooled on the cooling rolls (set to a temperature of 25°C), and solidified, thereby bonding the two spunbond nonwoven fabrics to the water-vapor permeable polyurethane film without using an adhesive.
As described above, a laminate where the first spunbond nonwoven fabric, the water-vapor permeable polyurethane film, and the second spunbond nonwoven fabric were sequentially laminated was prepared.
Thereafter, the laminate was left to stand in a constant temperature and humidity room at 23°C and 55% relative humidity for a week. Next, five sheets each having a size of 200 mm in the machine direction (MD) × 200 mm in the cross direction (CD) were cut out of the laminate.
Then, the sheets were stretched by a batch stretching apparatus under the conditions of a tension rate of 300 mm/min and a stretching ratio in the CD direction of 3.0 times, and immediately recovered to the original length, thereby preparing laminate sheets.

<<Example 2>>

Except that the following points were changed, a laminate sheet was prepared in the same manner as Example 1.

- The water-vapor permeable polyurethane A of Preparation Example 1 was replaced with the water-vapor permeable polyurethane B of Preparation Example 2.
- The temperature of the T die at the tip of the extruder was changed to 210°C.
- The temperature of each cylinder of the extruder was changed to that of Preparation Example 2.

<<Example 3>>

Except that the following points were changed, a laminate sheet was prepared in the same manner as Example 1.

- The water-vapor permeable polyurethane A of Preparation Example 1 was replaced with the water-vapor permeable polyurethane C of Preparation Example 3.
- The temperature of the T die at the tip of the extruder was changed to 218°C.
- The temperature of each cylinder of the extruder was changed to that of Preparation Example 3.

<<Example 4>>

Except that the following points were changed, a laminate sheet was prepared in the same manner as Example 1.

- The water-vapor permeable polyurethane A of Preparation Example 1 was replaced with the water-vapor permeable polyurethane D of Preparation Example 4.
- The temperature of the T die at the tip of the extruder was changed to 210°C.
- The temperature of each cylinder of the extruder was changed to that of Preparation Example 4.

<<Example 5>>

Except that the following points were changed, a laminate sheet was prepared in the same manner as Example 1.

- The water-vapor permeable-polyurethane A of Preparation Example 1 was replaced with the water-vapor permeable polyurethane E of Preparation Example 5.
- The temperature of the T die at the tip of the extruder was changed to 218°C.
- The temperature of each cylinder of the extruder was changed to that of Preparation Example 5.

<<Example 6>>

Except that the following points were changed, a laminate sheet was prepared in the same manner as Example 1.

- The water-vapor permeable polyurethane A of Preparation Example 1 was replaced with the water-vapor permeable polyurethane F of Preparation Example 6.
- The temperature of the T die at the tip of the extruder was changed to 218°C.
- The temperature of each cylinder of the extruder was changed to that of Preparation Example 6.

«Example 7»

Except that the following points were changed, a laminate sheet was prepared in the same manner as Example 1.

- The water-vapor permeable polyurethane A of Preparation Example 1 was replaced with the water-vapor permeable polyurethane G of Preparation Example 7.
- The temperature of the T die at the tip of the extruder was changed to 218°C.
- The temperature of each cylinder of the extruder was changed to that of Preparation Example 7.

<<Example 8>>

Except that the following points were changed, a laminate sheet was prepared in the same manner as Example 1.

- The water-vapor permeable polyurethane A of Preparation Example 1 was replaced with the water-vapor permeable polyurethane H of Preparation Example 8.
- The temperature of the T die at the tip of the extruder was changed to 218°C.
- The temperature of each cylinder of the extruder was changed to that of Preparation Example 8.

<<Example 9>>

Except that the following points were changed, a laminate sheet was prepared in the same manner as Example 1.

- The PO/TPU spunbond nonwoven fabric of Preparation Example 15 was replaced with the stretched and recovered TPU spunbond nonwoven fabric of Preparation Example 18.
- The stretching by the batch stretching apparatus was not carried out after the laminating step.

<<Comparative Example 1>>

Except that the following points were changed, a laminate sheet was prepared in the same manner as Example 1.

- The water-vapor permeable polyurethane A of Preparation Example 1 was replaced with the water-vapor permeable polyurethane I of Preparation Example 9.
- The PO/TPU spunbond nonwoven fabric of Preparation Example 15 was replaced with the PO spunbond nonwoven fabric of Preparation Example 16.
- The temperature of the T die at the tip of the extruder was changed to 223°C.
- The temperature of each cylinder of the extruder was changed to that of Preparation Example 9.

<<Comparative Example 2>>

The water-vapor permeable polyurethane J of Preparation Example 10 and the PO spunbond nonwoven fabric of Preparation Example 16 were set in the melt extrusion laminator illustrated in FIG. 2. However, the water-vapor permeable polyurethane J was not molten as describe in Preparation Example 10, and thus the preparation of a laminate sheet failed.

<<Comparative Example 3»

The water-vapor permeable polyurethane K of Preparation Example 11 and the PO spunbond nonwoven fabric of Preparation Example 16 were set in the melt extrusion laminator illustrated in FIG. 2. However, the water-vapor permeable polyurethane K was not solidified as describe in Preparation Example 11, and thus the preparation of a laminate sheet failed.

<<Comparative Example 4»

Except that the following points were changed, a laminate sheet was prepared in the same manner as Example 1.

- The water-vapor permeable polyurethane A of Preparation Example 1 was replaced with the water-vapor permeable polyurethane L of Preparation Example 12.
- The temperature of the T die at the tip of the extruder was changed to 210°C.
- The temperature of each cylinder of the extruder was changed to that of Preparation Example 12.

<<Comparative Example 5>>

Except that the following points were changed, a laminate sheet was prepared in the same manner as Example 1.

- The water-vapor permeable polyurethane A of Preparation Example 1 was replaced with the water-vapor permeable polyurethane M of Preparation Example 13.
- The temperature of the T die at the tip of the extruder was changed to 227°C.
- The temperature of each cylinder of the extruder was changed to that of Preparation Example 13.

<<Comparative Example 6>>

Except that the following points were changed, a laminate sheet was prepared in the same manner as Example 1.

- The PO/TPU spunbond nonwoven fabric of Preparation Example 15 was replaced with the PO spunbond nonwoven fabric of Preparation Example 16.
- The temperature of the T die at the tip of the extruder was changed to 218°C.

<<Comparative Example 7>>

Except that the following points were changed, a laminate sheet was prepared in the same manner as Example 1.

- The PO/TPU spunbond nonwoven fabric of Preparation Example 15 was replaced with the TPU spunbond nonwoven fabric of Preparation Example 17.
- The temperature of the T die at the tip of the extruder was changed to 218°C.

<<Comparative Example 8>>

Except that the following points were changed, a laminate sheet was prepared in the same manner as Example 1.

- The water-vapor permeable polyurethane A of Preparation Example 1 was replaced with the water-vapor permeable polyurethane I of Preparation Example 9.
- The temperature of the T die at the tip of the extruder was changed to 223°C.
- The temperature of each cylinder of the extruder was changed to that of Preparation Example 9.

<Evaluations of Laminate Sheet>

<<Peeling Strength of Spunbond Nonwoven Fabric (adhesion, unit: N)>>

A test piece with a size of a width of 50 mm × a length of 200 mm was collected from the laminate sheet of each of Examples and Comparative Examples. Next, a part of one of the two spunbond nonwoven fabrics was peeled from the water-vapor permeable polyurethane film to expose the water-vapor permeable polyurethane film. Next, an end of the peeled spunbond nonwoven fabric and an end of the exposed water-vapor permeable polyurethane film were held by the chucks (at a holding interval therebetween of 50 mm) to measure the peeling strength of the spunbond nonwoven fabric at a tension rate of 200 mm/min. Five maximum load points were obtained and the average value was calculated as the measurement value. The results are shown in Tables 1 and 2.

«Stretchability of Laminate Sheet (Unit: %)»

A test piece with a size of a width of 50 mm × a length of 200 mm was collected from the laminate sheet of each of Examples and Comparative Examples. Next, both ends in the length direction of the test piece were held by the chucks (at a holding interval therebetween of 100 mm), and the test piece was stretched at a tension rate of 300 mm/min and a stretching ratio of 100%, and was immediately recovered to the original length at the same rate to measure the strain at the recovery as the residual strain (%). Five residual strain points in the MD direction were obtained and the average was calculated as the residual strain. The results are shown in Tables 1 and 2.

<<Dynamic Coefficient of Friction of Laminate Sheet (Tackiness Properties, Unit: N/A)>>

A test piece with a size of a width of 150 mm × a length of 400 mm was collected from the laminate sheet of each of Examples and Comparative Examples. Then, the test piece was set on the test surface and a slip piece (nonwoven fabric) of 100 mm per side was also set on the test surface so that the spunbond nonwoven fabric of the test piece was in contact with the slip piece in conformity to JIS K7125. Next, the slip piece was slipped at a tension rate of 200 mm/min and traveled a travel distance of 200 mm to record the load. The dynamic coefficient of friction was obtained from a chart. The measurement was carried out three times and the average value was calculated. The results are shown in Tables 1 and 2.

<<Water-vapor Permeability of Laminate Sheet (Unit: g/$m^2$. 24 h)>>

The water-vapor permeability of the laminate sheet of each of Examples and Comparative Examples was measured based on the method specified by JIS L 1099 A-1 under the conditions of a temperature of 40°C, a relative humidity of 90%, and use of calcium chloride. 10 samples were randomly collected and the average value was calculated. The measurement was carried out for a measurement time of two hours, and the measurement value for the two hours was converted to that for 24 hours. The results are shown in Tables 1 and 2.

[Table 1]

| No. | | | | | Example 1 | Example 2 | Example 3 | Example 4 | Example 5 | Example 6 | Example 7 | Example 8 | Example 9 |
|---|---|---|---|---|---|---|---|---|---|---|---|---|---|
| Formulation [parts by mass] | Spunbond nonwoven fabric | | | | PO/TPU | PO/TPU | PO/TPU | PO/TPU | PO/TPU | PO/TPU | PO/TPU | PO/TPU | PO/TPU* |
| | Water-vapor permeable polyurethane | | | | A | B | C | D | E | F | G | H | A |
| | Film | Polyisocyanate | | MDI | 329.26 | 229.43 | 377.8 | 162.88 | 193.91 | 201.66 | 304.74 | 190.9 | 329.26 |
| | | Polyol | Polyether polyol | A (Mn=1950) | 604.9 | 604.9 | 609.4 | | | | | | 604.9 |
| | | | | B (Mn=995) | 159.15 | 159.15 | 159.15 | | | | | | 159.15 |
| | | | | C (Mn=2270) | | | | 702.67 | | | | | |
| | | | | D (Mn=2945) | | | | | | | 912.91 | | |
| | | | | E (Mn=997) | | | | | | | | 309.17 | |
| | | | Polycarbonate polyol | F (Mn=1993) | | | | | 617.8 | | | | |
| | | | Polycaprolactone polyol | G (Mn=2008) | | | | | | 622.22 | | | |
| | | | Chain extender | 1,4-BD | 74.78 | 39.64 | 91.9 | 30.18 | 41.45 | 44.15 | 82.4 | 40.27 | 74.78 |
| | | | | NPG | | | | | | | | | |
| | | Antioxidant | | | 2.92 | 2.58 | 3.1 | 2.24 | 2.13 | 2.17 | 3.25 | 1.35 | 2.92 |
| | | Ultraviolet absorber | | | 1.75 | 1.55 | 1.86 | 1.34 | 1.28 | 1.3 | 1.95 | 0.81 | 1.75 |
| | | Lubricant | | | 0.58 | 0.52 | 0.62 | 0.45 | 0.43 | 0.43 | 0.65 | 0.27 | 0.58 |
| Evaluation | | Hard segment concentration (%) | | | 24.2 | 14.5 | 28.0 | 12.7 | 18.3 | 19.2 | 23.9 | 28.1 | 24.2 |
| | | Melting point of hard segment phase (°C) | | | 104 | 78 | 131 | 95 | 131 | 134 | 131 | 118 | 104 |
| | Laminate | Peeling strength (N) | | | 0.7 | 0.6 | 0.6 | 0.5 | 0.5 | 0.5 | 0.5 | 0.5 | 1.2 |
| | | Stretchability (%) | | | 5 | 6 | 7 | 10 | 13 | 14 | 15 | 15 | 13 |
| | | Dynamic coefficient of friction | | | 0.2 | 0.2 | 0.2 | 0.2 | 0.2 | 0.2 | 0.2 | 0.3 | 0.2 |
| | | Water-vapor permeability ($g/m^2 \cdot 24h$) | | | 6150 | 6020 | 5950 | 6020 | 1030 | 1120 | 5450 | 5100 | 6070 |

* Stretched in stretching process

[Table 2]

| | | No. | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|---|---|
| Formulation [parts by mass] | | Spunbond nonwoven fabric | PO | PO | PO | PO/TPU | PO/TPU | PO | TPU | PO/TPU |
| | | Water-vapor permeable polyurethane | I | J | K | L | M | A | A | I |
| | Film | Isocyanate MDI | 349.03 | 304.74 | 304.74 | 176.39 | 458.62 | 329.26 | 329.26 | 349.03 |
| | Polyol | Polyether polyol A (Mn=1950) | | | | 604.9 | 604.9 | 604.9 | 604.9 | |
| | | Polyether polyol B (Mn=995) | | | | 159.15 | 159.15 | 159.15 | 159.15 | |
| | | Polyether polyol C (Mn=2270) | | | | | | | | |
| | | Polyether polyol D (Mn=2945) | 912.91 | 912.91 | 912.91 | | | | | 912.91 |
| | | Polyether polyol E (Mn=997) | | | | | | | | |
| | | Polycarbonate polyol F (Mn=1993) | | | | | | | | |
| | | Polycaprolactone polyol G (Mn=2008) | | | | | | | | |
| | | Chain extender 1,4-BD | 96.41 | 55.05 | | 20.72 | 120.37 | 74.78 | 74.78 | 96.41 |
| | | Chain extender NPG | | | 137.42 | | | | | |
| | | Antioxidant | 3.4 | 3.18 | 3.39 | 2.4 | 3.36 | 2.92 | 2.92 | 3.4 |
| | | Ultraviolet absorber | 2.04 | 1.91 | 2.03 | 1.44 | 2.01 | 1.75 | 1.75 | 2.04 |
| | | Ethylene bis stearic acid amide | 0.68 | 0.68 | 0.68 | 0.48 | 0.67 | 0.58 | 0.58 | 0.68 |
| | | Hard segment concentration (%) | 26.8 | 16.3 | 34.5 | 8.1 | 33.8 | 24.2 | 24.2 | 26.8 |
| | | Melting point of hard segment phase (°C) | 145 | Water-vapor permeable polyurethane was not molten and thus formation of laminated sheet failed | Water-vapor permeable polyurethane was not molten and thus formation of laminated sheet failed | 62 | 150 | 104 | 104 | 145 |
| Evaluation | Laminate | Peeling strength (N) | Peeling occurred and thus measurement failed | | | 0.6 | 0.3 | 0.1 | 0.7 | 0.3 |
| | | Stretchability (%) | Peeling occurred and thus measurement failed | | | 14 | 21 | 28 | 4 | 21 |
| | | Dynamic coefficient of friction | 0.2 | | | 0.7 | 0.2 | 0.2 | 0.8 | 0.3 |
| | | Water-vapor permeability (g/m$^2$·24h) | 4810 | | | 6370 | 4820 | 4350 | 6450 | 5215 |

While the illustrative embodiments of the present invention are provided in the above description, such is for illustrative purpose only and it is not to be construed as limiting in any manner. Modification and variation of the present invention that will be obvious to those skilled in the art is to be covered by the following claims.

Industrial Applicability

The laminate sheet, sanitary material, medical material, and method for manufacturing a laminate sheet of the present invention are suitable for domestic use, medical and sanitary use, industrial use, civil engineering and architectural use, agricultural and horticultural use, and clothing use.

Description of Reference Numerals

1 laminate sheet
2 water-vapor permeable polyurethane film
3 first spunbond nonwoven fabric
4 second spunbond nonwoven fabric

## Claims

**1.** A laminate sheet comprising:

a water-vapor permeable polyurethane film having a hard segment phase; and
a first spunbond nonwoven fabric disposed at one side in a thickness direction of the water-vapor permeable polyurethane film, the first spunbond nonwoven fabric containing stretchable fibers containing a thermoplastic polyurethane and non-stretchable fibers containing a polyolefin, wherein
the hard segment phase has a melting point of 65°C or more and 140°C or less.

**2.** The laminate sheet according to claim 1, wherein

the water-vapor permeable polyurethane film has a soft segment phase, and
the soft segment phase is formed by a reaction of a polyether polyol and a polyisocyanate.

**3.** The laminate sheet according to claim 2, wherein
the polyether polyol has a number average molecular weight of 1200 g/mol or more and 2800 g/mol or less.

**4.** The laminate sheet according to claim 1, further comprising:
a second spunbond nonwoven fabric disposed at the other side in the thickness direction of the water-vapor permeable polyurethane film, the second spunbond nonwoven fabric containing stretchable fibers containing a thermoplastic polyurethane and non-stretchable fibers containing a polyolefin.

**5.** The laminate sheet according to claim 1, wherein
the first spunbond nonwoven fabric is in direct contact with a one-side surface in the thickness direction of the water-vapor permeable polyurethane film.

**6.** A sanitary material comprising the laminate sheet according to claim 1.

**7.** A medical material comprising the laminate sheet according to claim 1.

**8.** A method for manufacturing a laminate sheet, the method comprising:

a step of mixing and joining stretchable fibers containing a thermoplastic polyurethane and non-stretchable fibers containing a polyolefin by heat fusion to prepare a spunbond nonwoven fabric;
a step of preparing a water-vapor permeable polyurethane film having a hard segment phase, the hard segment phase having a melting point of 65°C or more and 140°C or less;
a step of applying pressure on the spunbond nonwoven fabric against the water-vapor permeable polyurethane film to laminate the spunbond nonwoven fabric and the water-vapor permeable polyurethane film; and

a step of subjecting the spunbond nonwoven fabric to a stretching process before the laminating step; and/or
a step of subjecting the laminated spunbond nonwoven fabric and water-vapor permeable polyurethane film to a stretching process after the laminating step.

**9.** The method according to claim 8, wherein
the spunbond nonwoven fabric is in direct contact with a one-side surface in a thickness direction of the water-vapor permeable polyurethane film in the step of laminating the spunbond nonwoven fabric and the water-vapor permeable polyurethane film.

FIG. 1

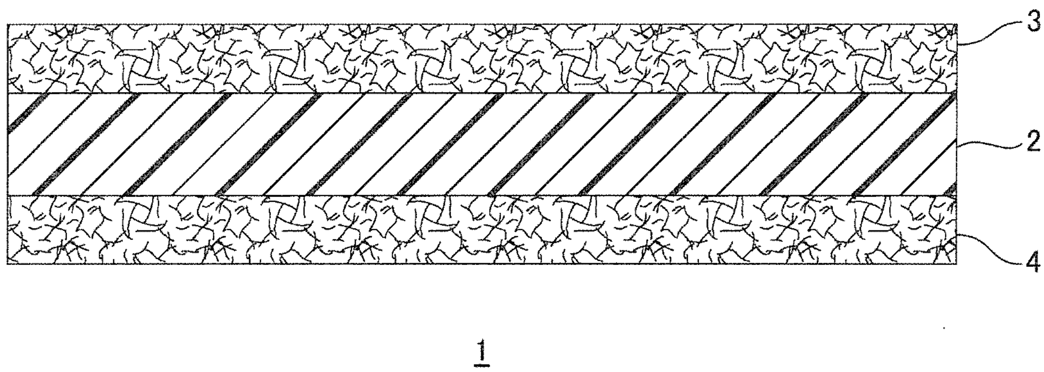

FIG. 2

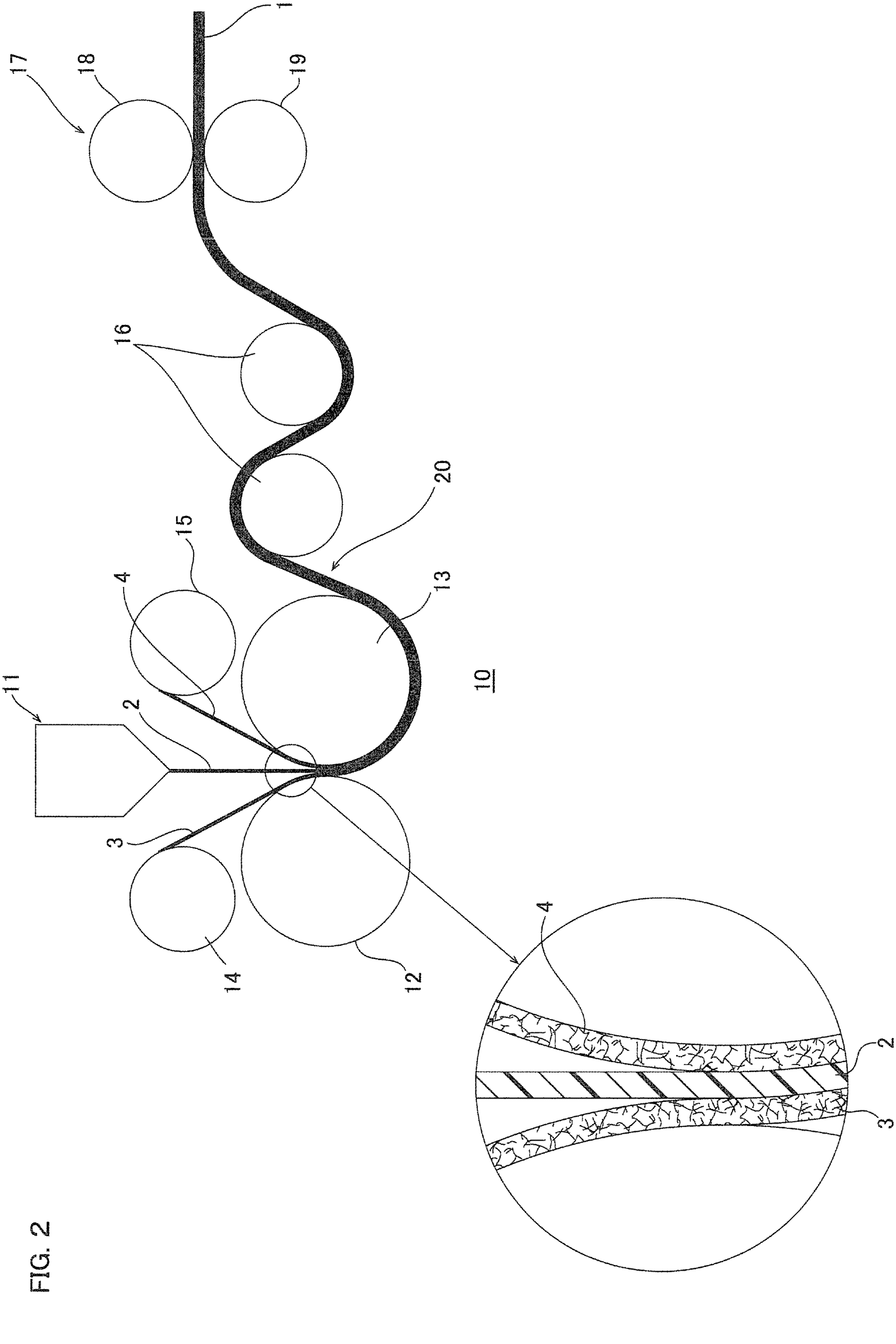

## INTERNATIONAL SEARCH REPORT

International application No.
PCT/JP2021/012252

A. CLASSIFICATION OF SUBJECT MATTER
Int. Cl. B32B27/40(2006.01)i, A61F13/51(2006.01)i, A61L15/24(2006.01)i, A61L15/26(2006.01)i, A61L15/42(2006.01)i, B32B5/02(2006.01)i, B32B27/12(2006.01)i, B32B27/32(2006.01)i
FI: B32B27/40, A61F13/51, A61L15/24 100, A61L15/26 100, A61L15/42 100, B32B5/02 A, B32B27/12, B32B27/32 Z

According to International Patent Classification (IPC) or to both national classification and IPC

B. FIELDS SEARCHED

Minimum documentation searched (classification system followed by classification symbols)
Int. Cl. B32B1/00-43/00, A61L15/00-15/64, C08J5/00-5/02, C08J5/12-5/22, C08G18/00-18/87, C08G71/00-71/04, A61F13/51

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2008-213284 A (MITSUI CHEMICALS, INC.) 18 September 2008, claims 1, 2, 6-8, 11, paragraphs [0059]-[0061] | 1-9 |
| A | WO 2008/108238 A1 (MITSUI CHEMICALS, INC.) 12 September 2008, claims 1, 2, 6, 12, paragraphs [0077], [0078], [0085]-[0098], [0173]-[0175] | 1-9 |
| A | JP 7-285187 A (NEW OJI PAPER CO., LTD.) 31 October 1995, claim 1, paragraph [0001] | 1-9 |
| A | JP 9-286085 A (KAO CORP.) 04 November 1997, claims 1, 2, 6, paragraph [0001] | 1-9 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19.05.2021 | 01.06.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/012252

C (Continuation). DOCUMENTS CONSIDERED TO BE RELEVANT

| Category* | Citation of document, with indication, where appropriate, of the relevant passages | Relevant to claim No. |
|---|---|---|
| A | JP 2017-95681 A (SANYO CHEMICAL INDUSTRIES, LTD.) 01 June 2017, claims, examples, paragraph [0063] | 1-9 |
| A | JP 2010-6882 A (NISSHINBO HOLDINGS INC.) 14 January 2010, claims, paragraph [0013] | 1-9 |

Form PCT/ISA/210 (continuation of second sheet) (January 2015)

**INTERNATIONAL SEARCH REPORT**
Information on patent family members

International application No.
PCT/JP2021/012252

| Patent Documents referred to in the Report | Publication Date | Patent Family | Publication Date |
|---|---|---|---|
| JP 2008-213284 A | 18.09.2008 | (Family: none) | |
| WO 2008/108238 A1 | 12.09.2008 | US 2010/0105273 A1<br>claims 1, 2, 6, 12, paragraphs [0078], [0079], [0086]-[0099]<br>EP 2116367 A1<br>CN 101622124 A<br>KR 10-2009-0117829 A | |
| JP 7-285187 A | 31.10.1995 | US 5624424 A<br>claim 1, column 1, lines 5-13<br>KR 10-1995-0024750 A | |
| JP 9-286085 A | 04.11.1997 | (Family: none) | |
| JP 2017-95681 A | 01.06.2017 | (Family: none) | |
| JP 2010-6882 A | 14.01.2010 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H770936 A
- JP 4332627 B
- JP 2008213284 A
- JP 2004244791 A
- JP H11106500 A